# EUROPEAN PATENT APPLICATION

(11) **EP 4 159 199 A1**
(43) Date of publication of application: **05.04.2023**
(21) Application number: 21199872.9
(22) Date of filing: 29.09.2021
(51) Int. Cl.: A61K 9/16, A61K 9/20, A61K 31/426, A61K 31/4725, A61K 45/06, A61K 9/24, A61K 9/48, A61P 13/00

(54) **COMBINED FORMULATION OF MIRABEGRON AND SOLIFENACIN**

(71) Applicant: Lotus Pharmaceutical Co., Ltd., 11046 Taipei City (TW)
(72) Inventor: Mehta, Gaurav Bhupendrabhai, Nantou City (TW); Gupta, Vijender, Nantou City (TW); Chawla, Manish, Nantou City (TW)
(74) Representative: Hartvichova, Katerina

(57) **Abstract**

The present invention provides a combined pharmaceutical form comprising
- a first component containing modified-release mirabegron or a pharmaceutically acceptable salt thereof, preferably extended-release mirabegron or a pharmaceutically acceptable salt thereof, and
- a second component containing immediate-release solifenacin or a pharmaceutically acceptable salt thereof.

## Description

### Field of Art

The present invention relates to a pharmaceutical combination formulation comprising mirabegron or a pharmaceutically acceptable salt thereof and solifenacin or a pharmaceutically acceptable salt thereof.

### Background Art

Co-administration of mirabegron ((R)-2-(2-aminothiazol-4-yl)-4'-{2-[(2-hydroxy-2-phenylethyl)amino]ethyl} acetanilide) and solifenacin ((3R)-quinuclidin-3-yl (1S)-1-phenyl-1,2,3,4-tetrahydroisoquinoline-2-carboxylate) to treat urinary urgency, urinary frequency and/or urge urinary incontinence associated with overactive bladder is known in the art (WO2009057685).

EP 2891493 discloses a pharmaceutical combination formulation comprising both actives, which is a multi-layered composition (bi- or tri-layered tablet or a coated tablet wherein the core contains mirabegron and the coating contains solifenacin, para 0076, but only bi-layer tablets are disclosed in the examples and tested). The modified release part comprises mirabegron and contains a hydrogel-forming polymer (polyethylene oxide) and an additive allowing water to penetrate into the modified release portion. Furthermore, inclusion of calcium stearate into the solifenacin layer is recommended as otherwise the release rate of solifenacin is negatively affected in the combination product.

EP 3448367 teaches the use of solifenacin succinate and at least 50 wt.% of a water insoluble diluent in the immediate release part of the combination product.

None of the proposed combination products has been launched to the market yet. There is still a need to provide an improved combined preparation which would be suitable for practical use.

### Disclosure of the Invention

The present invention relates to a pharmaceutical form comprising a first component containing modified-release mirabegron or a pharmaceutically acceptable salt thereof, preferably extended-release mirabegron or a pharmaceutically acceptable salt thereof, and a second component containing immediate-release solifenacin or a pharmaceutically acceptable salt thereof.

Immediate release is a formulation which releases and delivers a drug immediately after its administration, without delaying or prolonging dissolution or absorption of the drug.

Modified release is a formulation that (in contrast to immediate-release dosage) delivers a drug with a delay after its administration (delayed-release dosage) or for a prolonged period of time (extended-release [ER, XR, XL] dosage) or to a specific target in the body (targeted-release dosage).
Extended release is a type of the modified release formulations. Extended-release dosage is either sustained-release (SR) or controlled-release (CR) dosage. SR maintains drug release over a sustained period but not at a constant rate.

In a first aspect of the invention, the pharmaceutical form is a capsule filled with tablets or minitablets or granules containing a modified-release formulation of mirabegron or a pharmaceutically acceptable salt thereof, preferably an extended-release formulation of mirabegron or a pharmaceutically acceptable salt thereof, and with tablets or minitablets or granules of an immediate-release formulation of solifenacin or a pharmaceutically acceptable salt thereof.

Oral-solid dosage (OSD) drugs can be formulated in tablet or capsule form.

Minitablets are tablets having a diameter within the range from 1.5 mm to 6 mm, particularly preferably from 2 mm to 5 mm, especially preferably about 5 mm.

Various types of capsules, with shells made of different materials are available, e.g. hard gelatin, HPMC, fish gelatin, starch, pullulan, polyvinyl acetate capsules.
Hard gelatin capsules is a routinely used type of capsules. The gelatin used in the manufacture of most common capsules is obtained from collagenous material by hydrolysis. Gelatin is a natural, safe, non-allergenic, clean, and economical ingredient.
HPMC capsules are made of hydroxypropyl methyl cellulose (HPMC) and are stable at low humidity levels, have low moisture content (3-8%), and low static charge.
Fish gelatin capsules, or marinecaps, are made from fish gelatin.
Starch capsules are made from potato starch. Their dissolution is pH independent.
Pullulan capsules are suitable also for vegetarians. They are made from tapioca, which is naturally fermented into pullulan. They provide a high barrier to oxygen.
Polyvinyl acetate (PVA) capsules are made from a synthetic polymer PVA. The oxygen permeability of PVA is low, resulting in a high barrier to oxygen.

In a second aspect of the invention, the pharmaceutical form is a tablet having a core containing or consisting of a modified-release formulation of mirabegron or a pharmaceutically acceptable salt thereof or an extended-release formulation of mirabegron or a pharmaceutically acceptable salt thereof, said core being coated by a coating containing or consisting of an immediate-release formulation of solifenacin or a pharmaceutically acceptable salt thereof.

Further aspects of the invention may include multilayer tablets comprising a layer containing or consisting of a modified-release formulation of mirabegron or a pharmaceutically acceptable salt thereof, preferably an extended-release formulation of mirabegron or a pharmaceutically acceptable salt thereof, and a layer containing or consisting of an immediate-release formulation of solifenacin or a pharmaceutically acceptable salt thereof, and optionally a separation layer free of active pharmaceutical ingredient; or a capsule filled with tablets or granules containing or consisting of a modified-release formulation of mirabegron or a pharmaceutically acceptable salt thereof, preferably an extended-release formulation of mirabegron or a pharmaceutically acceptable salt thereof, and with tablets or granules containing or consisting of an immediate-release formulation of solifenacin or a pharmaceutically acceptable salt thereof.

The granules or tablets may be uncoated or film-coated.

In some embodiments of the invention, the modified-release formulation of mirabegron or a pharmaceutically acceptable salt thereof comprises mirabegron or a pharmaceutically acceptable salt thereof as an active ingredient, a release-controlling agent polyethylene oxide and at least one pharmaceutically acceptable excipient. Polyethylene oxide may include polyethylene oxide with an average molecular weight of 100,000 to 900,000 and/or polyethylene oxide with an average molecular weight of 2 million to 8 million.
In some embodiments, polyethylene oxide with an average molecular weight of about 300,000, and/or polyethylene oxide with an average molecular weight of about 7 million is used.
Preferably, polyethylene oxide with the average molecular weight of 100,000 to 900,000 is included in the amount of 30 to 90%, preferably 40 to 75% by weight or 40 to 65% by weight, based on the total weight of the mirabegron-containing composition.
Preferably, polyethylene oxide with the average molecular weight of about 300,000 is included in the amount of 30 to 90%, preferably 40 to 75%, most preferably 42 to 61% by weight, based on the total weight of the mirabegron-containing composition.
In some embodiments, the pharmaceutical composition may include as a release-controlling agent a polyethylene oxide with an average molecular weight of 100,000 to 900,000 and polyethylene oxide with an average molecular weight of 2 million to 8 million at the same time. It is preferred for polyethylene oxide with the average molecular weight of 100,000 to 900,000 to be in an amount of 30 to 90, more preferably 40 to 75% by weight based on the total weight of the mirabegron-containing composition., and polyethylene oxide with the average molecular weight of 2 million to 8 million is preferably in an amount of 1 to 30%, more preferably 2 to 20% by weight, based on the total weight of the mirabegron-containing composition.
In one preferred embodiment, the mirabegron-containing composition comprises as a the release-controlling agent polyethylene oxide with an average molecular weight of 300,000 in an amount of 43-45% and polyethylene oxide with an average molecular weight of 7 million in an amount of 4-5%, based on the total weight of the mirabegron-containing composition.
If the amount of a polyethylene oxide exceeds the above limits, the release of mirabegron is excessively inhibited, resulting in low absorption into the body. If the amount of a polyethylene oxide is below the lower limit, the release of mirabegron may be excessively dependent on the presence or absence of the intake of food.

Further pharmaceutically acceptable excipients in the pharmaceutical composition of the invention may include an antioxidant, a diluent and a lubricant.

The antioxidant may be selected from butylhydroxytoluene (BHT), propyl gallate (PG), butylhydroxyanisole (BHA), ascorbic acid, sodium ascorbate, erythorbic acid, sodium nitrite, sodium hydrogen sulfite, sodium pyrosulfite, and citric acid. A particularly preferred antioxidant is butylhydroxytoluene (BHT). The antioxidant may preferably be present in the amount of 0.01 to 2% by weight based on the total weight of the mirabegron-containing composition.

The lubricant is preferably present in the amount of 0.05 to 4% by weight based on the total weight of the mirabegron-containing composition. The lubricant may be selected from the group of compounds consisting of magnesium stearate, talc, hydrogenated castor oil, calcium stearate and silicon dioxide, and, preferably, magnesium stearate.

The diluent is preferably present in the amount of 10 to 25 % by weight based on the total weight of the mirabegron-containing composition to ensure uniform flow, desirable compression properties, and content uniformity. The diluent may be selected from the group consisting of silicified microcrystalline cellulose or cellulose microcrystalline, low substituted hydroxypropyl cellulose, starch, and calcium phosphate, and preferably silicified microcrystalline cellulose.

The mirabegron-containing composition of the present invention preferably does not include hydrophilic materials capable of attracting water inside the mirabegron-containing composition, i.e., hydrophilic materials which allow water to penetrate into the mirabegron-containing composition. Such hydrophilic materials capable of attracting water inside the mirabegron-containing composition or allowing water to penetrate into the mirabegron-containing composition are hydrophilic materials having a solubility such that the amount of water necessary to dissolve 1 g of the hydrophilic material is 10 ml or less.

The mirabegron-containing composition is preferably produced using a dry granulation process, i.e., substantially without using any granulating solvents such as water or ethanol.

In some embodiments the immediate-release formulation of solifenacin or a pharmaceutically acceptable salt thereof contains at least one hydrophilic substance selected from the group consisting of D-mannitol, maltose, polyethylene glycol (for example, product names PEG 400, PEG 1500, PEG 4000, PEG 6000, and PEG 20000) and polyvinylpyrrolidone. The at least one hydrophilic substance may be used as a filler and/or a binder.
The amount of the at least one hydrophilic substance in the composition is 2 - 95 % by weight, preferably 10 - 60 % by weight, or 30 - 50 % by weight, relative to the solifenacin-containing formulation. Furthermore, the immediate-release formulation of solifenacin or a pharmaceutically acceptable salt thereof contains lactose monohydrate in order to improve the release rate of solifenacin.
The amount of lactose in the composition is 2 - 95 % by weight, preferably 10 - 60 % by weight, or 30 - 50 % by weight, relative to the solifenacin-containing formulation.

A particularly preferred pharmaceutically acceptable salt of solifenacin is solifenacin succinate.

The combined formulation of the present invention shows independent release of mirabegron and solifenacin, thus, there is no mutual influencing of the release of pharmaceutically active ingredients.

### Examples

### Example 1: Film coated tablet comprising Mirabegron

Formulation process for modified release preparation: Drug binder dispersion with ethanol was prepared, granulation was done using high shear granulator or fluid bed processor with polyethylene oxide (PEO) 300K (WSRN750 NF) polymer and with silicified microcrystalline cellulose, the amounts of polymer and silicified microcrystalline cellulose are in 1:1 ratio. Butylated hydroxytoluene (BHT) was added, which acts as an antioxidant in the formulation and helps to stabilize the composition. Magnesium stearate in the formulation acts as lubricant. Tablets were compressed and coated by Opadry yellow. (Opadry contains Hypromellose, iron oxide yellow and macrogol)

| Material Name | Mg/tablet | %w/w |
|---|---|---|
| Dispersion | | |
| Mirabegron | 50.00 | 15.87 |
| Hydroxypropyl cellulose | 15.00 | 4.76 |
| Purified water | q.s. | q.s. |

| Intragranular - Material | | |
|---|---|---|
| Silicified Microcrystalline cellulose (SMCC) | 120.00 | 38.10 |
| PEO 300 K (WSR N-750 NF) | 120.00 | 38.10 |
| BHT | 0.40 | 0.13 |
| Magnesium Stearate | 2.60 | 0.83 |
| Core Tablet weight | 308.00 | 97.78 |

| Coating material | | |
|---|---|---|
| Ethanol: Purified water (80:20) | q.s. | q.s. |
| Opadry Yellow | 7.00 | 2.22 |
| Total weight of tablet | 315.00 | 100.00 |

### Example 2: Film coated tablet comprising Mirabegron

Film coated tablets were prepared by direct mixing process - simple co-sifting and mixing all the intragranular excipients followed by compression and hydroalcoholic coating.

| Material Name | Mg/tablet | %w/w |
|---|---|---|
| Mirabegron | 50.00 | 17.67 |
| Cellulose microcrystalline (MCC) | 80.10 | 28.30 |
| PEO 300 K (WSR N-750 NF) | 143.00 | 50.53 |
| BHT | 0.40 | 0.14 |
| Magnesium Stearate | 2.50 | 0.88 |
| Core Tablet weight | 276.00 | 97.53 |

| Coating material | | |
|---|---|---|
| Ethanol: Purified water (80:20) | q.s. | q.s. |
| Opadry Yellow | 7.00 | 2.47 |
| Total weight of tablet | 283.00 | 100.00 |

### Example 3: Film coated tablet comprising Mirabegron

Film coated tablets were prepared by dry granulation process - co-sifting, pre-mixing of all the intragranular excipients, then dry granulation by using roller compactor followed by compression and non-aqueous coating.

| Material Name | Mg/tablet | %w/w |
|---|---|---|
| Mirabegron | 50.00 | 19.01 |
| Cellulose microcrystalline (MCC) | 120.50 | 45.82 |
| PEO 300 K (WSR N-750 NF) | 82.00 | 31.18 |
| BHT | 2.00 | 0.76 |
| Magnesium Stearate | 1.50 | 0.57 |
| Core Tablet weight | 256.00 | 97.34 |

| Coating material | | |
|---|---|---|
| Ethanol | q.s. | q.s. |
| Opadry Yellow | 7.00 | 2.66 |
| Total weight of tablet | 263.00 | 100.00 |

### Example 4: Film coated tablets comprising Mirabegron

Formulation process for modified release preparation: the hydrophilic polymer with microcrystalline cellulose were co-sifted, amounts of polymer and insoluble excipients are in approximately 1: 1.7 ratio. Butylated hydroxytoluene (BHT) was co-sifted, which acts as an antioxidant in the formulation and helps to stabilize the composition. Magnesium stearate added in the formulation acts as a lubricant. Dry granulation was performed by using roller compactor, followed by milling and final blending. Tablets were compressed and coated by using the Opadry yellow. (Opadry contains hypromellose, iron oxide yellow and macrogol)

| Material Name | Mg/tablet | %w/w |
|---|---|---|
| Mirabegron | 50.00 | 18.38 |
| Cellulose microcrystalline (MCC) | 80.10 | 29.45 |
| PEO 300 K (WSR N-750 NF) | 120.00 | 44.12 |
| PEO 7 M (WSR-303 NF) | 13.00 | 4.78 |
| BHT | 0.40 | 0.15 |
| Magnesium Stearate | 1.50 | 0.55 |
| Core Tablet weight | 265.00 | 97.43 |

| Coating material | | |
|---|---|---|
| Ethanol | q.s. | q.s. |
| Opadry Yellow | 7.00 | 2.57 |
| Total weight of tablet | 272.00 | 100.00 |

### Example 5: Film coated minitablets comprising Mirabegron

Formulation process for modified release preparation: the polymers we co-sifted with cellulose microcrystalline (MCC). Butylated hydroxytoluene was added during the co-sifting process, and acts as an antioxidant in the formulation and helps to stabilize the composition. Magnesium stearate was added to the formulation and acts as a lubricant.
Dry granulation was performed by using Roller compactor, followed by milling and final blending. Tablets were compressed and coated by using Opadry yellow. (Opadry contains hypromellose, iron oxide yellow and macrogol.)

| Material Name | Mg/tablet | %w/w |
|---|---|---|
| Mirabegron | 5.00 | 18.38 |
| Cellulose microcrystalline (MCC) | 8.01 | 29.45 |
| PEO 300 K (WSR N-750 NF) | 12.00 | 44.12 |
| PEO 7 M (WSR-303 NF) | 1.30 | 4.78 |
| BHT | 0.04 | 0.15 |
| Magnesium Stearate | 0.15 | 0.55 |
| Core Tablet weight | 26.50 | 97.43 |

| Coating material | | |
|---|---|---|
| Ethanol | q.s. | q.s. |
| Opadry Yellow | 0.70 | 2.57 |
| Total weight of tablet | 27.20 | 100.00 |

### Example 6: Film coated minitablets comprising Mirabegron

Formulation process for modified release preparation is by co-sifting the polymers with cellulose microcrystalline (MCC). Butylated hydroxytoluene (BHT) was added during the co-sifting process, which acts as an antioxidant in the formulation and helps to stabilize the composition. Magnesium stearate was added, which acts in the formulation as a lubricant.
Dry granulation was performed by using Roller compactor, followed by milling and final blending. Tablets were compressed and coatied by using Opadry yellow. (Opadry contains Hypromellose, iron oxide yellow and macrogol.)

| Material Name | Mg/tablet | %w/w |
|---|---|---|
| Mirabegron | 10.00 | 18.38 |
| Cellulose microcrystalline (MCC) | 16.02 | 29.45 |
| PEO 300 K (WSR N-750 NF) | 24.00 | 44.12 |
| PEO 7 M (WSR-303 NF) | 2.60 | 4.78 |
| BHT | 0.08 | 0.15 |
| Magnesium Stearate | 0.30 | 0.55 |
| Core Tablet weight | 53.00 | 97.43 |

| Coating material | | |
|---|---|---|
| Ethanol | q.s. | q.s. |
| Opadry Yellow | 1.40 | 2.57 |
| Total weight of tablet | 54.40 | 100.00 |

### Example 7: Immediate release formulation comprising Solifenacin succinate

The granules were prepared by a wet granulation process by mixing the solifenacin succinate, mannitol, lactose monohydrate and L HPC LH 11. The components were granulated using purified water as a granulating solvent. The granules were lubricated with magnesium stearate.
The granules were further compressed into tablets which were coated by using Opadry orange. (Opadry contains hypromellose, iron oxide yellow, iron oxide red and macrogol.)
Immediate release film coated tablets comprising 5 mg of Solifenacin succinate contain:

| Material Name | Mg/tablet | %w/w |
|---|---|---|
| Solifenacin Succinate | 5.00 | 6.80 |
| L HPC LH 11 | 5.00 | 6.80 |
| Mannitol | 30.00 | 40.82 |
| Lactose monohydrate | 30.00 | 40.82 |
| Purified water | q.s. | q.s. |
| Magnesium stearate | 1.00 | 1.36 |
| Total weight of tablet | 71.00 | 96.60 |

| Film coat | | |
|---|---|---|
| Opadry Orange | 2.50 | 3.40 |
| Purified water | q.s. | - |
| Total weight of Tablet | 73.50 | 100.00 |

### Example 8: Immediate release film coated minitablets comprising 1 mg of Solifenacin succinate

Wet granulation process was performed by mixing the solifenacin succinate, mannitol, lactose monohydrate and L HPC LH 11 and granulating the mixture by using purified water as a granulating solvent. Granules were lubricated with magnesium stearate, and further compressed into tablets which were coated by using Opadry orange. (Opadry contains hypromellose, iron oxide yellow, iron oxide red and macrogol.)

| Material Name | Mg/tablet | %w/w |
|---|---|---|
| Solifenacin Succinate | 1.00 | 6.80 |
| L HPC LH 11 | 1.00 | 6.80 |
| Mannitol | 6.00 | 40.82 |
| Lactose monohydrate | 6.00 | 40.82 |
| Purified water | q.s. | q.s. |
| Magnesium stearate | 0.20 | 1.36 |
| Total weight of tablet | 14.20 | 96.60 |

| Film coat | | |
|---|---|---|
| Opadry Orange | 0.50 | 3.40 |
| Purified water | q.s. | - |
| Total weight of Tablet | 14.70 | 100.00 |

### Example 9: Immediate release film coated minitablets comprising 2.5 mg of Solifenacin succinate

Wet granulation process was performed by mixing the solifenacin succinate, mannitol, lactose monohydrate and L HPC LH 11, granulating the mixture using purified water as a granulating solvent. The granules were lubricated with magnesium stearate, and further compressed into minitablets and coated using Opadry orange. (Opadry contains hypromellose, iron oxide yellow, iron oxide red and macrogol.)

| Material Name | Mg/tablet | %w/w |
|---|---|---|
| Solifenacin Succinate | 2.50 | 6.80 |
| L HPC LH 11 | 2.50 | 6.80 |
| Mannitol | 15.00 | 40.82 |
| Lactose monohydrate | 15.00 | 40.82 |
| Purified water | q.s. | q.s. |
| Magnesium stearate | 0.50 | 1.36 |
| Total weight of tablet | 35.50 | 96.60 |

| Film coat | | |
|---|---|---|
| Opadry Orange | 1.25 | 3.40 |
| Purified water | q.s. | - |
| Total weight of Tablet | 36.75 | 100.00 |

### Example 10: Immediate release granules containing Solifenacin succinate wherein the granules are film coated

Wet granulation process was performed by mixing the solifenacin succinate, mannitol, lactose monohydrate and L HPC LH 11, and granulating the mixture using purified water as a granulating solvent. The granules were lubricated with magnesium stearate, compressed and coated using Opadry orange. (Opadry contains Hypromellose, iron oxide yellow, iron oxide red and macrogol.)

| Material Name | mg/granles | %w/w |
|---|---|---|
| Solifenacin Succinate | 5.00 | 6.80 |
| L HPC LH 11 | 5.00 | 6.80 |
| Mannitol | 30.00 | 40.82 |
| Lactose monohydrate | 30.00 | 40.82 |
| Purified water | q.s. | q.s. |
| Magnesium stearate | 1.00 | 1.36 |
| Total weight of granules | 71.00 | 96.60 |

| Film coat | | |
|---|---|---|
| Opadry Orange | 2.50 | 3.40 |
| Purified water | q.s. | - |
| Total weight of granules | 73.50 | 100.00 |

### Example 11: Multilayer tablet

| Material Name | Mg/tablet | %w/w |
|---|---|---|
| Mirabegron layer | | |
| Mirabegron | 50.00 | 13.77 |
| Silicified Microcrystalline cellulose (SMCC) | 80.10 | 22.18 |
| PEO 300 K (WSRN-750 NF) | 140.00 | 38.57 |
| BHT | 1.00 | 0.28 |
| Magnesium Stearate | 2.50 | 0.69 |
| Mirabegron layer weight | 274.00 | 75.48 |

| Separation layer | | |
|---|---|---|
| HPMC | 5.00 | 1.38 |
| PEG 600 | 2.00 | 0.55 |
| Ethanol: Purified water (80:20) | q.s. | - |
| Talc | 2.00 | 0.55 |

| Solifenacin layer | | |
|---|---|---|
| Solifenacin Succinate | 5.00 | 1.38 |
| HPC L | 5.00 | 1.38 |
| Mannitol | 30.00 | 8.26 |
| Lactose monohydrate | 30.00 | 8.26 |
| Purified water | q.s. | - |
| Total weight of tablet core | 353.00 | 97.25 |

| Film coat | | |
|---|---|---|
| Opadry Yellow | 10 | 2.75 |
| Ethanol: Purified water (80:20) | q.s. | - |
| Total weight of tablet | 363.00 | 100.00 |

The above formulation in the form of a multilayer coated tablet contains immediate release drug layer for solifenacin succinate and an modified-release layer for mirabegron.
Formulation process for modified release preparation: Co-sift the hydrophilic polymer with silicified microcrystalline cellulose. The amounts of hydrophilic polymer and insoluble excipients are in approximately 1:1.5 ratio. Co-sift butylated hydroxytoluene, which acts as an antioxidant in the formulation and helps to stabilize the composition. Add magnesium stearate into the formulation as a lubricant. Dry granulation was performed by using Roller compactor, followed by milling and final blending. Tablets with suitable weight and coating by using barrier coat material. Tablets further coated by immediate release drug dispersion/solution of solifenacin succinate layer. Finally, tablets coated by using the Opadry yellow. (Opadry contains Hypromellose, iron oxide yellow and macrogol).

### Example 12: Hard capsule containing 5mg IR Solifenacin succinate + 50 mg SR Mirabegron

Encapsulation performed for modified release tablets of mirabegron (Example 3) and immediate release tablets of solifenacin succinate (Example 7) in HPMC capsules shell size 0.

| Material Name | mg/capsule | %w/w |
|---|---|---|
| Solifenacin succinate tablet (Example 7) | 73.50 | 16.99 |
| Mirabegron prolonged-release tablet (Example 3) | 263.00 | 60.81 |
| HPMC capsule shell Size "0" | 96.00 | 22.20 |
| Total weight of filled capsule | 432.50 | 100.00 |

Dissolution tests for both active ingredients were perfomed as follows:
The dissolution testing conditions for Mirabegron are the following:

| Parameters | Dissolution Tester Conditions |
|---|---|
| Medium | pH 6.8 potassium phosphate buffer, 900 mL |
| Apparatus | USP I (Basket) + JP sinker |
| Rotation | 100 rpm |
| Temperature | 37.0 ± 0.5°C |
| Sampling Time | 1,3,4,8,10 hours |
| Sampling Volume | 10 mL |

The results of comparative dissolution tests are the following:

| Time points (Hrs) | Betmiga^{™} prolonged release tablets 50 mg | Test product Example 12 |
|---|---|---|
| Dissolution condition | % drug release in pH 6.8 potassium phosphate buffer using USP I (Basket) + JP sinker, (Sinker inside basket) at 100 rpm | |
| 1 hr | 9 | 10 |
| 3 hr | 34 | 37 |
| 4 hr | 48 | 49 |
| 8 hr | 92 | 82 |
| 10 hr | 101 | 93 |

Mirabegron dissolution in USP buffer, pH 6.8, paddle with 50 rpm in 900 ml volume shows less than 30 % drug release after 1 hr, and more than 80% drug release after 8 hrs, which is similar to dissolution of Betmiga^{™} 50 mg prolonged-release tablets.

The dissolution testing conditions for Solifenacin are the following:

| Parameters | Dissolution Tester Conditions |
|---|---|
| Medium | pH 6.8 potassium phosphate buffer, 900 mL |
| Apparatus | USP II Paddle, 50 RPM, 900 ml |
| Rotation | 50 rpm |
| Temperature | 37.0 ± 0.5°C |
| Sampling Time | 10, 15, 30, 45 mins - for Solifenacin Succinate |
| Sampling Volume | 10 mL |

The results of the comparative dissolution tests are the following:

| Time points (min) | Vesicare^{™} immediate release tablets 5 mg | Test product Example 12 |
|---|---|---|
| Dissolution condition | % drug release in pH 6.8 potassium phosphate buffer using USP II, 50 RPM, 900 ml | |
| 5 | 26 | 25 |
| 10 | 53 | 52 |
| 15 | 72 | 71 |
| 30 | 91 | 90 |
| 45 | 95 | 94 |

Solifenacin dissolution in USP II, buffer pH 6.8, paddle with 50 RPM in 900 ml volume shows more than 70% drug release after 15 mins, which is similar to dissolution of Vesicare^{™} 5 mg immediate-release tablet.

The above results show independent release of Mirabegron and Solifenacin from the formulation according to the invention.

Surprisingly we found in the present invention when both the drug products are separately filled in the capsule formulation will not retard the drug or impact the drug release of immediate release formulation or prolonged release formulation.

### Example 13: Capsule filled with Mirabegron prolonged-release minitablets + Solifenacin tablet

| Material Name | mg/capsule | %w/w |
|---|---|---|
| Mirabegron prolonged-release minitablets 25 mg Example 5 | 136.00 (5 minitablets) | 44.52 |
| Solifenacin succinate film coated tablet 5 mg Example 7 | 73.50 | 24.06 |
| Gelatin capsule shell Size "0" | 96.00 | 31.42 |
| Total weight of filled capsule | 305.50 | 100.00 |

### Example 14: Capsule filled with Mirabegron prolonged-release tablet + Solifenacin tablet

| Material Name | mg/capsule | %w/w |
|---|---|---|
| Mirabegron prolonged-release tablet 50 mg Example 4 | 272.00 | 61.61 |
| Solifenacin succinate film coated tablet 5 mg Example 7 | 73.50 | 16.65 |
| Gelatin capsule shell Size "0" | 96.00 | 21.74 |
| Total weight of filled capsule | 441.50 | 100.00 |

### Example 15: Capsule filled with Mirabegron prolonged-release tablet + Solifenacin minitablets

| Material Name | mg/capsule | %w/w |
|---|---|---|
| Mirabegron prolonged-release tablet 50 mg Example 4 | 272.00 | 61.61 |
| Solifenacin succinate film coated minitablets 5 mg Example 8 | 73.50 (5 minitablets) | 16.65 |
| Gelatin capsule shell Size "0" | 96.00 | 21.74 |
| Total weight of filled capsule | 441.50 | 100.00 |

### Example 16: Capsule filled with Mirabegron prolonged-release tablet + Solifenacin minitablets

| Material Name | mg/capsule | %w/w |
|---|---|---|
| Mirabegron prolonged-release tablet 50 mg Example 4 | 272.00 | 52.82 |
| Solifenacin succinate film coated minitablets 10 mg Example 9 | 147.00 (4 minitablets) | 28.54 |
| Gelatin capsule shell Size "0" | 96.00 | 18.64 |
| Total weight of filled capsule | 515.00 | 100.00 |

### Example 17: Capsule filled with Mirabegron prolonged-release minitablets + Solifenacin film coated minitablets

| Material Name | mg/capsule | %w/w |
|---|---|---|
| Mirabegron prolonged-release minitablets 25 mg Example 5 | 136.00 (5 minitablets) | 35.88 |
| Solifenacin succinate film coated minitablets 10 mg Example 9 | 147.00 (4 minitablets) | 38.79 |
| Gelatin capsule shell Size "0" | 96.00 | 25.33 |
| Total weight of filled capsule | 379.00 | 100.00 |

### Example 18: Capsule filled with Mirabegron prolonged-release minitablets + Solifenacin film coated minitablets

| Material Name | mg/capsule | %w/w |
|---|---|---|
| Mirabegron prolonged-release minitablets 50 mg Example 6 | 272.00 (5 minitablets) | 52.82 |
| Solifenacin succinate film coated minitablets 10 mg Example 9 | 147.00 (4 minitablets) | 28.54 |
| Gelatin capsule shell Size "0" | 96.00 | 18.64 |
| Total weight of filled capsule | 515.00 | 100.00 |

### Example 19: Capsule filled with Mirabegron prolonged-release minitablets + Solifenacin film coated minitablets

| Material Name | mg/capsule | %w/w |
|---|---|---|
| Mirabegron prolonged-release minitablets 50 mg Example 6 | 272.00 (5 minitablets) | 61.61 |
| Solifenacin succinate film coated minitablets 5 mg Example 9 | 73.50 (2 minitablets) | 16.65 |
| Gelatin capsule shell Size "0" | 96.00 | 21.74 |
| Total weight of filled capsule | 441.50 | 100.00 |

## Claims

1. A pharmaceutical form comprising
- a first component containing modified-release mirabegron or a pharmaceutically acceptable salt thereof, preferably extended-release mirabegron or a pharmaceutically acceptable salt thereof, and
- a second component containing immediate-release solifenacin or a pharmaceutically acceptable salt thereof.

2. The pharmaceutical form according to claim 1, wherein the pharmaceutical form is a capsule filled with tablets or minitablets or granules containing a modified-release formulation of mirabegron or a pharmaceutically acceptable salt thereof, preferably an extended-release formulation of mirabegron or a pharmaceutically acceptable salt thereof, and with tablets or minitablets or granules of an immediate-release formulation of solifenacin or a pharmaceutically acceptable salt thereof.

3. The pharmaceutical form according to claim 1, wherein the pharmaceutical form is a tablet having a core containing or consisting of a modified-release formulation of mirabegron or a pharmaceutically acceptable salt thereof or an extended-release formulation of mirabegron or a pharmaceutically acceptable salt thereof, said core being coated by a coating containing or consisting of an immediate-release formulation of solifenacin or a pharmaceutically acceptable salt thereof.

4. The pharmaceutical form according to claim 1, wherein the pharmaceutical form is a multilayer tablet comprising a layer containing or consisting of a modified-release formulation of mirabegron or a pharmaceutically acceptable salt thereof, preferably an extended-release formulation of mirabegron or a pharmaceutically acceptable salt thereof, and a layer containing or consisting of an immediate-release formulation of solifenacin or a pharmaceutically acceptable salt thereof, and optionally a separation layer free of active pharmaceutical ingredient.

5. The pharmaceutical form according to any one of claims 1 to 4, wherein the modified-release formulation of mirabegron or a pharmaceutically acceptable salt thereof comprises mirabegron or a pharmaceutically acceptable salt thereof as an active ingredient, a release-controlling agent polyethylene oxide and at least one pharmaceutically acceptable excipient, wherein the polyethylene oxide may includes polyethylene oxide with an average molecular weight of 100,000 to 900,000 and/or polyethylene oxide with an average molecular weight of 2 million to 8 million.

6. The pharmaceutical form according to claim 5, wherein polyethylene oxide with the average molecular weight of 100,000 to 900,000 is included in the amount of 30 to 90%, preferably 40 to 75% by weight or 40 to 65% by weight, based on the total weight of the mirabegron-containing composition.

7. The pharmaceutical form according to claim 5, wherein polyethylene oxide with the average molecular weight of 100,000 to 900,000 is included in an amount of 30 to 90, more preferably 40 to 75% by weight based on the total weight of the mirabegron-containing composition, and polyethylene oxide with the average molecular weight of 2 million to 8 million is included in an amount of 1 to 30%, more preferably 2 to 20% by weight, based on the total weight of the mirabegron-containing composition.

8. The pharmaceutical form according to claim 5, which further contains a diluent in the amount of 10 to 25 % by weight based on the total weight of the mirabegron-containing composition, wherein the diluent is selected from the group consisting of silicified microcrystalline cellulose, cellulose microcrystalline, low substituted hydroxypropyl cellulose, starch, and calcium phosphate; preferably silicified microcrystalline cellulose.

9. The pharmaceutical form according to claim 5, wherein the mirabegron-containing composition is free of hydrophilic materials which allow water to penetrate into the mirabegron-containing composition.

10. The pharmaceutical form according to any one of claims 1 to 9, wherein the immediate-release formulation of solifenacin or a pharmaceutically acceptable salt thereof contains at least one hydrophilic substance selected from the group consisting of D-mannitol, maltose, polyethylene glycol and polyvinylpyrrolidone.

11. The pharmaceutical form according to claim 10, wherein the amount of the at least one hydrophilic substance in the composition is 2 ― 95 % by weight, preferably 10 - 60 % by weight, or 30 - 50 % by weight, relative to the solifenacin-containing formulation.

12. The pharmaceutical form according to claim 10 or 11, wherein the immediate-release formulation of solifenacin or a pharmaceutically acceptable salt thereof contains lactose monohydrate, preferably in an amount of 2 - 95 % by weight, preferably 10 - 60 % by weight, or 30 - 50 % by weight, relative to the solifenacin-containing formulation.

13. The pharmaceutical form according to any one of claims 1 to 12, wherein the tablets or minitablets or granules are film-coated.

14. The pharmaceutical form according to any one of claims 1 to 13 for use in the treatment of urinary urgency, urinary frequency and/or urge urinary incontinence associated with overactive bladder.
